(19) Europäisches Patentamt
European Patent Office
Office européen des brevets

(11) Veröffentlichungsnummer: **0 038 479**
**B1**

(12) # EUROPÄISCHE PATENTSCHRIFT

(45) Veröffentlichungstag der Patentschrift: **09.02.83**

(51) Int. Cl.³: **B 01 J 27/18**, B 01 J 37/00, C 07 C 11/02, C 07 C 2/18

(21) Anmeldenummer: **81102696.2**

(22) Anmeldetag: **09.04.81**

(54) **Katalysatoren für die Oligomerisierung von Olefinen.**

(30) Priorität: **18.04.80 DE 3014950**

(43) Veröffentlichungstag der Anmeldung: **28.10.81 Patentblatt 81/43**

(45) Bekanntmachung des Hinweises auf die Patenterteilung: **09.02.83 Patentblatt 83/6**

(84) Benannte Vertragsstaaten: **BE DE FR GB IT NL**

(56) Entgegenhaltungen:
**DE-C-881 495**
**FR-A-1 374 756**
**US-A-2 237 822**
**UA-A-2 494 510**
**US-A-2 620 364**
**CHEMICAL ABSTRACTS, Band 87, Nr. 7, 15. August 1977, Seite 419, Nr. 52732f Columbus, Ohio, U.S.A.**

(73) Patentinhaber: **BASF Aktiengesellschaft, Carl-Bosch-Strasse 38, D-6700 Ludwigshafen (DE)**

(72) Erfinder: **Engelbach, Heinz, Dr., Kropsburgstrasse 24, D-6703 Limburgerhof (DE)**
Erfinder: **Steigleiter, Werner, Im Waldhof 4, D-6703 Limburgerhof (DE)**
Erfinder: **Glietenberg, Helmut, Dr., Thomas-Mann-Strasse 70, D-6700 Ludwigshafen (DE)**

EP 0 038 479 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Katalysatoren für die Oligomerisierung von Olefinen

Die vorliegende Erfindung betrifft neue Katalysatoren auf der Basis von Bor-, Antimon-, Wismut-, Aluminium- oder Titanphosphat oder Mischphosphaten dieser Elemente sowie ein Verfahren zur Oligomerisierung von Olefinen unter Verwendung dieser Katalysatoren.

Es ist allgemein bekannt, z. B. aus der Monographie von E. G. Hancock, »Propylene and its Industrial Derivatives«, Ernest Benn Verlag, London, 1973, S. 138 ff., Propen unter erhöhtem Druck und bei erhöhten Temperaturen an phosphorsäurehaltigen Trägerkatalysatoren zum Trimeren, Tetrameren und zu höheren Oligomeren zu oligomerisieren. Auf prinzipiell gleiche Weise können auch, wie ebenfalls allgemein bekannt ist, beispielsweise die Butene oligomerisiert werden.

Sowohl der Grundtyp der hierfür verwendeten Katalysatoren, mit Phosphorsäure imprägniertes $SiO_2$, als auch zahlreiche Varianten mit weiteren Komponenten wie Schwefel- und Fluorverbindungen (US-A-3 244 767 bzw. US-A-3 426 089) befriedigen im Hinblick auf die Lebensdauer und auf die Selektivität bezüglich der vornehmlich gewünschten Oligomeren, nämlich von Trimerpropenen und Dimerbutenen, nicht. Vor allem bei hohen Olefinumsätzen läßt die Selektivität zu wünschen übrig, d. h. man erhält Gemische von Oligomeren verschiedenen Oligomerisationsgrades mit hohen Anteilen an unerwünschten Oligomeren.

Auch die $H_3PO_4/SiO_2$-Trägerkatalysatoren gemäß der FR-A-1 374 756, bei denen ein Teil des $SiO_2$ durch andere Oxide, darunter Bortrioxid, ersetzt ist, genügen den Ansprüchen nicht. Mit diesen Katalysatoren, bei denen das Molverhältnis von $P_2O_5$ zu $B_2O_3$ rund 9 : 1 bis 40 : 1 beträgt, lassen sich, wie eigene Versuche ergeben haben, Propenumsätze von etwa 90% erzielen, wobei die auf das erwünschte Wertprodukt, das trimere Propen, bezogenen Selektivitäten, also die auf den Propenumsatz bezogenen Ausbeuten, nur rund 75% betragen. Außerdem sinken die Umsätze bei Verwendung dieser Katalysatoren nach etwa einmonatigen Betriebszeiten auf 70—75% ab.

Es war daher allgemein Aufgabe der vorliegenden Erfindung, Katalysatoren für die Oligomerisierung von Olefinen zu entwickeln, mit denen sich über lange Betriebszeiten gleichbleibend hohe Umsätze erzielen lassen. Speziell betrifft diese Aufgabe Katalysatoren für die Oligomerisierung von Propen und von Butenen, und zwar mit dem zusätzlichen Erfordernis hoher Selektivitäten bezüglich Trimerpropenen bzw. von Dimerbutenen.

Es wurde gefunden, daß sich Mischverbindungen, welche der folgenden empirischen Formel I

$$APO_4 \cdot [P_2O_5 \cdot (H_2O)_n]_a \cdot (XY)_b \qquad (I)$$

entsprechen, in welcher die Variablen folgende Bedeutung haben

- A Bor, Antimon, Wismut, Aluminium und/oder 3 Äquivalente des Titans
- X $NH_4^{\oplus}$ und/oder ein Alkalimetallkation und/oder das Äquivalent eines Erdalkalimetallkations
- Y das Äquivalent eines Anions
- a 0,02—0,2
- b 0—0,05
- n 1—3

hervorragend als Katalysatoren für die Oligomerisierung von Olefinen eignen.

Unter diesen Katalysatoren werden diejenigen besonders bevorzugt, in denen A Bor bedeutet oder in denen der Anteil des Bors mindestens 80 Mol-% beträgt. Die Erfindung sei daher anhand der Katalysatoren auf der Basis von Borphosphat näher erläutert. Für die übrigen definitionsgemäßen Phosphate oder Mischphosphate gelten diese Erläuterungen sinngemäß.

Im einfachsten Falle bestehen diese Katalysatoren aus einer Mischverbindung aus $BPO_4$ einerseits und Orthophosphorsäure (n=3) oder einer Anhydrophosphorsäure (n=1 bis zu n=3 als Grenzfall) andererseits. Charakteristisch für diese Mischverbindungen ist, daß sie weitgehend, also etwa mindestens zu 95% des theoretisch möglichen Wertes die $BPO_4$-Struktur aufweisen, wie mit Hilfe des Röntgenbeugungsdiagramms ermittelt werden kann.

Man kann diese Katalysatoren herstellen, indem man wäßrige oder reine Phosphorsäure und Borsäure miteinander innig vermischt, diese Mischungen 2—30 Stunden lang unter Rühren und unter Rückflußkühlung bei 80—120°C hält und anschließend unter weitgehender Entfernung des nicht chemisch gebundenen Wassers solange auf etwa 80—120°C erhitzt, bis die Masse zähflüssig geworden ist. Danach wird sie bei 110—500°C getrocknet und dehydratisiert, pulverisiert, mit etwas Wasser zu einem Teig verknetet und in die gewünschte Form, z. B. Kugeln, Tabletten, Stränge oder Ringe gebracht. Die Stränge haben im allgemeinen einen Durchmesser von 1—20 mm und eine Länge von 2—40 mm. Für andere Formen gelten diese Dimensionen sinngemäß. Nach der Formgebung wird abermals getrocknet und dehydratisiert. Im Hinblick auf die mechanische Festigkeit der Katalysatorteilchen empfiehlt es sich, die Trocknung schonend vorzunehmen, etwa indem man die Teilchen zunächst 2—30 Stunden lang auf 80—120°C und danach 2—40 Stunden lang auf 120—500°C erhitzt.

Es ist auch möglich, die unter Borphosphatbildung verlaufende Vermischung der Borsäure und der Phosphorsäure bei geringeren Temperaturen, z. B. bei Raumtemperatur, und entsprechend längerer Zeit vorzunehmen. Ferner kann man die Formgebung auch mit der im Anschluß auf das erste Erhitzen erhältlichen Masse vornehmen, jedoch ist in diesem Falle die Borphosphatbildung meistens nicht ganz vollständig. Erhitzt man sehr hoch, so kann $P_2O_5$ sublimieren. Die hierdurch entstehenden Verluste können jedoch durch Zusatz entsprechender Mengen Phosphorsäure wieder ersetzt werden.

Selbstverständlich kann man anstelle der Phosphorsäure auch die anhydridischen Säuren wie Meta- und Pyrophosphorsäure sowie Polyphosphorsäuren und anstelle der Borsäure auch Bortrioxid einsetzen. Die Wirkung der Katalysatoren kann durch Zusätze gemäß dem Formelteil $(XY)_b$ weiter verbessert werden. Solche Zusätze X sind das Ammoniumkation sowie die Kationen der Alkalimetalle und die Kationäquivalente der Erdalkalimetalle. Bevorzugt werden unter diesen Metallen Na, K und Ca. Man stellt diese Katalysatoren auf analoge Weise wie die zusatzfreien Katalysatoren her, indem man entsprechende Mengen von Salzen XY, wobei Y Anionäquivalente sind, mitverwendet. Auf die Natur der Anionen kommt es nach den bisherigen Beobachtungen nicht an, so daß man z. B. die Nitrate, Sulfate oder Chloride einsetzen kann. Bevorzugt werden jedoch die Borate und Phosphate oder Salze, z. B. Carbonate oder Formiate, die bei der Katalysatorherstellung in die Borate oder Phosphate übergehen. Ist Y Borat oder Phosphat, so ist es als $B_2O_3$ bzw. $P_2O_5$ dem Gesamt-$B_2O_3$ bzw. -$P_2O_5$ in der empirischen Formel I zuzurechnen. In diesem Falle wäre also Y der an B bzw. P gebundene anionische Sauerstoff.

Die chemische Konstitution der Katalysatoren ist im einzelnen nicht bekannt, jedoch ist ihre Wirksamkeit stets gegeben, wenn sie der empirischen Bruttoformel I entsprechen und wenn das Bor gemäß Röntgenbeugungsdiagramm mindestens zu 95% als Borphosphat vorliegt.

Vorzugsweise verwendet man die erfindungsgemäßen Katalysatoren als Substanzkatalysatoren. Man kann sie als aktive Masse nach den üblichen Techniken jedoch auch auf inerte Trägermaterialien wie $SiO_2$, $Al_2O_3$ oder Steatit aufbringen, wobei das Trägermaterial etwa 30 bis 95 Gew.-% des gesamten Trägerkatalysators betragen kann.

Die Oligomerisierung von Olefinen mit Hilfe der erfindungsgemäßen Katalysatoren kann diskontinuierlich oder vorzugsweise kontinuierlich in gasförmiger, flüssiger oder fluider Phase nach den üblichen Techniken und unter den üblichen Bedingungen erfolgen, also bei 50—300, vorzugsweise 100—200°C und 10—200, vorzugsweise 80—150 bar und mit dem reinen Olefin als Einsatzstoff oder vorzugsweise mit einer Mischung aus mindestens 10 Gew.-% des Olefins und dem Rest eines inerten Gases, vorzugsweise des entsprechenden Paraffins.

Für die bevorzugte kontinuierliche Oligomerisierung ordnet man den Katalysator zweckmäßigerweise als Festbett in freier Schüttung an und richtet den Olefindurchsatz so ein, daß pro Liter Katalysatorvolumen pro Stunde 0,25—6 l des flüssigen Olefins umgesetzt werden. Als Olefine eignen sich prinzipiell jegliche olefinisch ungesättigten polymerisierbaren Verbindungen, etwa $C_2$—$C_6$—$\alpha$-Alkene. Von besonderer praktischer Bedeutung ist hierbei die Trimerisierung von Propen und die Dimerisierung und Mischdimerisierung von But-1-en, But-2-en und Isobuten, wobei man ein Isomerengemisch von Nonenen bzw. Octenen erhält. Außerdem ist auch die Codimerisierung von Butenen mit Propen zu Heptenen erfolgreich möglich. Diese höheren Olefine lassen sich sodann durch Hydroformylierung und Hydrierung in Decanole bzw. Nonanole bzw. Octanole überführen, die ihrerseits zur Herstellung von Weichmachern und Detergentien dienen.

## Beispiele A—D

### Herstellung von Katalysatoren des Typs $BPO_4 \cdot [P_2O_5 \cdot (H_2O)_n]_a$

Zur Herstellung des Katalysators A wurden 1000 g reine Orthophosphorsäure, 176,5 g Wasser und 560 g Borsäure unter Rühren und Rückflußkühlung 15 Stunden lang auf 112°C erhitzt, wonach soviel Wasser abdestilliert wurde, daß eine zähe, breiartige Masse zurückblieb. Diese Masse wurde 15 Stunden lang bei 110°C und 24 Stunden lang bei 230°C getrocknet, pulverisiert, mit 465 g Wasser zu einem zähen Teig verknetet und zu Strängen von 4 mm Durchmesser und 4 mm Länge verpreßt. Diese Stränge wurden sodann 15 h bei 110°C und 24 h bei 230°C getrocknet.

In analoger Weise wurden die Katalysatoren B und C sowie der Vergleichskatalysator D hergestellt. Die Charakteristika dieser Katalysatoren sind der Tabelle 1 zu entnehmen.

## Beispiele E—N

### Herstellung von Katalysatoren des Typs $BPO_4 \cdot [P_2O_5 \cdot (H_2O)_n]_a \cdot (XY)_b$

Diese Katalysatoren wurden in gleicher Weise hergestellt wie die Katalysatoren A—D, jedoch mit dem Unterschied, daß ein Teil der Phosphorsäure durch Ammoniumdihydrogenphosphat bzw. ein

Alkalimetalldihydrogenphosphat bzw. ein Erdalkalihydrogenphosphat ersetzt wurde. Die Charakteristika dieser Katalysatoren sind ebenfalls der Tabelle 1 zu entnehmen.

Beispiele O—R

Herstellung von Katalysatoren des Typs $APO_4 \cdot [P_2O_5 \cdot (H_2O)_n]_a$

Diese Katalysatoren, die sich von den Katalysatoren A—N durch ein anderes Element A unterscheiden, wurden in analoger Weise wie die Katalysatoren A—N mit den Oxiden des Sb, Bi, Ti und Al hergestellt. Die Charakteristika dieser Katalysatoren sind ebenfalls der Tabelle 1 zu entnehmen.

Tabelle 1

Katalysatoren $APO_4 \cdot [P_2O_5 \cdot (H_2O)_n]_a \cdot (XO-)_b$

| Katalysator | A | a | n | X | b | Borphosphat nach Röntgenbeugungsdiagramm<br>% |
|---|---|---|---|---|---|---|
| A | Bor | 0,045 | 1,8 | — | — | in allen Fällen ≧95% |
| B | Bor | 0,063 | 1,8 | — | — | |
| C | Bor | 0,150 | 1,8 | — | — | |
| D, zum Vergleich | Bor | 0,250 | 1,8 | — | — | |
| E | Bor | 0,072 | 1,8 | $NH_4$ | 0,0071 | |
| F | Bor | 0,076 | 1,8 | $NH_4$ | 0,0125 | |
| G | Bor | 0,072 | 1,8 | $NH_4$ | 0,0214 | |
| H | Bor | 0,072 | 1,8 | $NH_4$ | 0,0285 | |
| I | Bor | 0,063 | 1,8 | K | 0,00625 | |
| K | Bor | 0,063 | 1,8 | K | 0,0125 | |
| L | Bor | 0,063 | 1,8 | Na | 0,0125 | |
| M | Bor | 0,063 | 1,8 | Rb | 0,0125 | |
| N | Bor | 0,063 | 1,8 | Ca | 0,00625 | |
| O | Sb | 0,025 | 1,8 | — | — | — |
| P | Bi | 0,063 | 1,8 | — | — | — |
| Q | Ti | 0,050 | 1,8 | — | — | — |
| R | Al | 0,063 | 1,8 | — | — | — |

Beispiele 1—18

Trimerisierung von Propen

Je 300 ml der Katalysatoren A—Q wurden in freier Schüttung in einen zylindrischen Versuchsreaktor von 3 cm Innendurchmesser und 2 m Länge eingebracht. In diesem Reaktor wurde ein Gemisch aus 50 Gew.-% Propan und 50 Gew.-% Propen mit einer Katalysatorbelastung von 0,5 l flüssigem Propen pro Liter Katalysatorvolumen und pro Stunde unter den aus Tabelle 2 ersichtlichen weiteren Reaktionsbedingungen der auf die Trimerisierung ausgerichteten Oligomerisierung von Propen unterworfen, wobei sich die angegebenen Werte auf den stationären Betrieb beziehen. Die Temperatur war jeweils die Temperatur des Kühlmediums (RMarlotherm), welches den Reaktor umgab und dessen Temperatur seinerseits mittels Wasserkühlung konstant gehalten wurde.

Die Verfahrensergebnisse, die nach den üblichen Analysemethoden wie Siedeanalyse, Gaschromatographie und Bestimmung der Hydrierjodzahl ermittelt wurden, sind ebenfalls der Tabelle 2 zu entnehmen. In allen Fällen entstanden neben den Nonenen hauptsächlich die Dodecene und geringe Mengen höherer Oligomerisate. Die Anteile an Hexenen sowie sonstiger Nebenprodukte waren vernachlässigbar.

In keinem Fall war innerhalb der Versuchszeit von 5 bis 30 Tagen ein Nachlassen der Katalysatoraktivität bezüglich Umsatz und Selektivität zu verzeichnen.

Tabelle 2

Trimerisierung von Propen zu Nonenen

| Beispiel | Kat. | Temp. | Druck | Propen-umsatz | Nonenausbeute bezogen auf Umsatz*) | Nonenausbeute absolut |
|---|---|---|---|---|---|---|
| | | °C | bar | % | % | % |
| 1 | A | 150 | 100 | 91 | 71 | 65 |
| 2 | B | 150 | 100 | 88 | 77 | 68 |
| 3 | C | 126 | 100 | 86 | 73 | 63 |
| 4**) | D**) | 130 | 100 | 92 | 54 | 50 |
| 5 | B | 140 | 150 | 88 | 76 | 67 |
| 6 | B | 140 | 100 | 89 | 74 | 66 |
| 7 | E | 140 | 100 | 84 | 79 | 66 |
| 8 | F | 150 | 100 | 86 | 77 | 66 |
| 9 | G | 175 | 100 | 87 | 90 | 78 |
| 10 | H | 195 | 100 | 80 | 86 | 69 |
| 11 | I | 150 | 100 | 84 | 74 | 62 |
| 12 | K | 175 | 100 | 84 | 87 | 73 |
| 13 | L | 150 | 100 | 84 | 81 | 68 |
| 14 | M | 140 | 100 | 88 | 74 | 65 |
| 15 | N | 150 | 100 | 85 | 78 | 66 |
| 16 | O | 140 | 100 | 89 | 72 | 64 |
| 17 | P | 190 | 100 | 42 | 83 | 35 |
| 18 | Q | 135 | 100 | 96 | 73 | 70 |

*) = Selektivität.
**) = zum Vergleich.

Beispiel 19

Dimerisierung von Butenen

24,3 g des Katalysators G, der eine Teilchengröße von 1—1,5 mm hatte, wurden in freier Schüttung in einen Versuchsreaktor von 8 mm inneren Durchmesser und 90 cm Höhe eingefüllt.

In diesem Reaktor wurde eine butenhaltige Kohienwasserstoff-Fraktion der folgenden Zusammensetzung

| | |
|---|---|
| But-1-en | 12,9 Vol.-% |
| cis-But-2-en | 23,0 Vol.-% |
| trans-But-2-en | 43,5 Vol.-% |
| Isobuten | 0,9 Vol.-% |

| | |
|---|---|
| Buta-1,3-dien | 0,03 Vol.-% |
| Propen | 0,73 Vol.-% |
| Butan | 15,7 Vol.-% |
| Isobutan | 3,1 Vol.-% |

bei einer stündlichen Katalysatorbelastung von 1,75 l flüssigen Butenen pro Liter Katalysatorvolumen bei 225° C und 90 bar der Dimerisierung unterworfen.

Der Butenumsatz betrug hier 82%, die Selektivität bezüglich der Dimeren 89% und die absolute Ausbeute hieran 73%. Diese Ergebnisse blieben über eine Beobachtungszeit von 10 Tagen konstant.

Beispiel 20

Dimerisierung von Butenen

Analog Beispiel 19, jedoch mit dem Katalysator R und einer Katalysatorbelastung von 0,28 l flüssigen Butenen pro Liter Katalysatorvolumen wurde das dort beschriebene Kohlenwasserstoffgemisch der Dimerisierungsreaktion unterworfen

Der Butenumsatz betrug 73%, die Selektivität bezüglich der Dimeren 74% und die absolute Ausbeute an Dimeren demgemäß 54%.

Beispiel 21

Analog Beispiel 19, jedoch mit dem Katalysator S

$BPO_4[P_2O_5 \cdot (H_2O)_{1,8}]_{0,072} \cdot [NH_4(SO_4)_{0,5}]_{0,0143}$, der wie die Katalysatoren E—N hergestellt wurde, sowie einer Katalysatorbelastung von 0,5 l flüssigen Butenen pro Liter Katalysatorvolumen und einer Temperatur von 180° C wurde das dort genannte Kohlenwasserstoffgemisch der Dimerisierungsreaktion unterworfen. Hierbei wurde ein Butenumsatz von 81% erzielt, und die Selektivität bezüglich des Dimeren betrug 88%. Dementsprechend fiel das Dimere in einer absoluten Ausbeute von 71% an.

**Patentansprüche**

1. Katalysatoren für die Oligomerisierung von Olefinen, deren aktive Masse der empirischen Formel I

$$APO_4 \cdot [P_2O_5 \cdot (H_2O)_n]_a \cdot (XY)_b \qquad (I)$$

entspricht, in welcher die Variablen folgende Bedeutung haben

A Bor, Antimon, Wismut, Aluminium und/oder 3 Äquivalente des Titans
X $NH_4^{\oplus}$ und/oder ein Alkalimetallkation und/oder das Äquivalent eines Erdalkalimetallkations
Y das Äquivalent eines Anions
a 0,02—0,2
b 0—0,05
n 1—3

2. Katalysatoren gemäß Anspruch 1, dadurch gekennzeichnet, daß das Element A Bor bedeutet.

3. Verfahren zur Herstellung der Katalysatoren I, dadurch gekennzeichnet, daß man wäßrige oder reine Phosphorsäure oder eine Anhydrophosphorsäure mit einem Oxid des Elementes A sowie gegebenenfalls einem Salz XY in den durch die Indices a und b gegebenen Mengenverhältnissen unter längerem Erwärmen innig vermischt, das überschüssige Wasser abdestilliert, bis man eine zähe bis feste Masse erhält, diese in die gewünschte Katalysatorform bringt und die Katalysatorteilchen soweit bei höheren Temperaturen trocknet, wie es dem gewünschten Gehalt n an gebundenem Wasser entspricht.

4. Verfahren nach Anspruch 3, dadurch gekennzeichnet, daß man die zunächst erhaltene zähe bis feste Masse bei höheren Temperaturen trocknet, die trockene Masse pulverisiert, mit Wasser anteigt, danach die Formgebung vornimmt und die Katalysatorteilchen bis zum gewünschten Gehalt n an gebundenem Wasser abermals trocknet.

5. Verfahren zur Oligomerisierung von Olefinen in an sich bekannter Weise, dadurch gekennzeichnet, daß man hierzu die Mischverbindungen I als Oligomerisierungskatalysatoren verwendet.

6. Verfahren nach Anspruch 5, dadurch gekennzeichnet, daß man hierzu die Katalysatoren gemäß Anspruch 2 (A = Bor) verwendet.

## Claims

1. A catalyst for olefin oligomerization, whose active material has the empirical formula I

$$APO_4 \cdot [P_2O_5 \cdot (H_2O)_n]_a \cdot (XY)_b \quad \text{(I)}$$

where A ist boron, antimony, bismuth, aluminium and/or 3 equivalents of titanium, X is $NH_4^{\oplus}$ and/or an alkali metal cation and/or the equivalent of an alkaline earth metal cation, Y is the equivalent of an anion, a is 0.02—0.2, b is 0—0.05 and n is 1—3.

2. A catalyst as claimed in claim 1, wherein element A is boron.

3. A process for the preparation of a catalyst I, wherein aqueous or pure phosphoric acid or an anhydrophosphoric acid is thoroughly mixed with an oxide of element A, with or without addition of a salt XY, in the ratios corresponding to the indices a and b, the mixture is heated for a lengthy period, the excess water is distilled off until a viscous or solid material is obtained, the latter ist brought to the desired catalyst shape and the catalyst particles are dried at an elevated temperature until the content of chemically bonded water corresponds to the desired value of n.

4. A process as claimed in claim 3, wherein the viscous or solid material first obtained is dried at an elevated temperature, the dry material is pulverized, the powder is worked into a paste with water, the paste is then molded and the catalyst particles are dried again until the content of chemically bonded water corresponds to the desired value of n.

5. A conventional olefin oligomerization process, wherein a mixed compound I is employed as the oligomerization catalyst.

6. A process as claimed in claim 5, wherein a catalyst as claimed in claim 2 (A = boron) is used.

## Revendications

1. Catalyseurs pour l'oligomérisation d'oléfines, dont la matière active correspond à la formule empirique I

$$APO_4 \cdot [P_2O_5 \cdot (H_2O)_n]_a \cdot (XY)_b \quad \text{(I)}$$

dans laquelle les variables possèdent les significations ci-après:

A = bore, antimoine, bismuth, aluminium et(ou) 3 équivalents du titane;
X = $NH_4^{\oplus}$ et(ou) un cation d'un métal alcalin et(ou) l'équivalent d'un cation d'un métal alcalino-terreux;
Y = l'équivalent d'un anion;
a = 0,02 à 0,2;
b = 0 à 0,05 et
n = à 3.

2. Catalyseurs suivant la revendication 1, caractérisés en ce que l'élément A est le bore.

3. Procédé de préparation de catalyseurs I, caractérisé en ce que l'on prépare, par un chauffage prolongé, un mélange intime d'acide phosphorique pur ou aqueux ou d'un acide anhydro-phosphorique et d'un oxyde d'un élément A, ainsi qu'éventuellement d'un sel XY, dans les proportions pondérales requises par les indices a et b, dont on élimine l'eau en excès par distillation de manière à obtenir une masse visqueuse à solide, que l'on façonne dans la forme voulue pour le catalyseur, les particules de catalyseur étant ensuite séchées à des températures accrues jusqu'à l'établissement de la teneur n en eau combinée souhaitée.

4. Procédé suivant la revendication 3, caractérisé en ce que la masse visqueuse à solide obtenue d'abord est séchée à des températures accrues, la masse séchée est pulvérisée, la poudre est transformée par addition d'eau en pâte qui est façonnée dans la forme voulue, puis les particules de catalyseur sont séchées une nouvelle fois jusqu'à l'établissement de la teneur n en eau combinée souhaitée.

5. Procédé d'oligomérisation d'oléfines, connue en soi, caractérisé en ce que l'on emploie comme catalyseurs d'oligomérisation des composés mixtes I.

6. Procédé suivant la revendication 5, caractérisé en ce que le catalyseur utilisé est un catalyseur selon la revendication 2 (A = bore).